# FASCICULE DE BREVET EUROPEEN

(11) **EP 2 528 556 B1**
(45) Date de publication et mention de la délivrance du brevet: **25.03.2015**
(21) Numéro de dépôt: 11706870.0
(22) Date de dépôt: 25.01.2011
(51) Int. Cl.: A61F 5/01

(54) **ORTHESE DU GENOU POUR RUPTURE DU LIGAMENT CROISE ANTERIEUR**
KNIEORTHESE FÜR GERISSENES VORDERES KREUZBAND
KNEE ORTHOSIS FOR TORN ANTERIOR CRUCIATE LIGAMENT

(30) Priorité: 26.01.2010 FR 1050518
(43) Date de publication de la demande: 05.12.2012
(73) Titulaire: Delin, Cyrille, 92100 Boulogne Billancourt (FR)
(72) Inventeur: Delin, Cyrille, 92100 Boulogne Billancourt (FR)
(74) Mandataire: Thivillier, Patrick
(86) Numéro de dépôt international: PCT/FR2011/050138
(87) Numéro de publication internationale: WO 2011/092423

(56) Documents cités:
- WO-A2-2009/149217
- DE-A1- 3 422 685
- DE-A1- 19 844 545
- US-A- 2 270 685
- US-A- 5 417 647

## Description

### DOMAINE TECHNIQUE DE L'INVENTION

L'invention concerne le domaine technique des orthèses, notamment pour le genou.

L'invention trouve une application particulièrement avantageuse pour les lésions traumatiques du ligament croisé antérieur.

### DESCRIPTION DE L'ART ANTERIEUR

On rappelle, d'une manière parfaitement connue pour un homme du métier, que le ligament croisé antérieur du genou, est tendu entre la partie antérieure du plateau tibial et la face interne du condyle fémoral externe du fémur. Ce ligament a pour fonction essentielle d'empêcher le glissement antérieur du tibia sous le fémur et également de contrôler la stabilité rotatoire du tibia sous le fémur, lors de la flexion du genou.

Une rupture du ligament croisé antérieur entraîne une instabilité du genou et, par conséquent, des risques de déboîtement. La thérapeutique d'une rupture du ligament croisé antérieur, peut aboutir à un traitement chirurgical ou à un traitement fonctionnel.

Le traitement chirurgical consiste en une ligamentoplastie du ligament croisé antérieur, c'est-à-dire à reconstruire le ligament, par exemple au moyen d'un greffon prélevé sur un tendon.

Le traitement fonctionnel consiste à suppléer l'absence du ligament croisé antérieur par une rééducation musculaire et proprioceptive pour permettre au genou de rester stable. Pour assurer cette stabilité du genou, il est très souvent nécessaire de porter une orthèse en particulier lors d'activités dites de « pivot » sollicitant particulièrement le ligament croisé antérieur, ce qui du reste peut également être le cas suite à un traitement chirurgical.

Différents types d'orthèses du genou sont actuellement disponibles.

Généralement, une orthèse comprend une armature articulée agencée pour être fixée au niveau de la cuisse et de la jambe, de part et d'autre du genou, avec des moyens de stabilisation de l'articulation du genou contre les forces varisantes et valgisantes. Par exemple, les armatures, au niveau de la cuisse et de la jambe, sont reliées par un système de tringles articulées avec des parties d'appui au niveau sensiblement du centre de rotation du genou.

On a proposé également des orthèses mettant en oeuvre un systèmede sangles comme il ressort de l'enseignement du document WO 2009/149217, qui décrit une orthèse selon le préambule de la revendication 1.

Il a toutefois été constaté que ces différents types d'orthèses ne permettent pas de réduire le tiroir antérieur qui existe en cas de rupture complète du ligament croisé antérieur.

L'invention s'est fixée pour but de remédier à ces inconvénients d'une manière simple, sûre, efficace et rationnelle.

### DESCRIPTION DE L'INVENTION

Le problème que se propose de résoudre l'invention est d'empêcher la partie antérieure et supérieure du tibia de glisser vers l'avant, en utilisant une orthèse.

Pour résoudre un tel problème, il a été conçu et mis au point une orthèse du type de celle définie dans la première partie de la revendication 1 et qui présente les caractéristiques relevant de la deuxième partie de la revendication 1.

Des résultats avantageux ont été obtenus avec quatre sangles convenablement positionnées au niveau de la jambe et de la cuisse, dans les conditions suivantes :
- Une première sangle est positionnée au niveau de la jambe et par rapport au centre de rotation du genou, à égale distance du plateau tibial et de la tubérosité tibiale antérieure et, au niveau de la cuisse, sensiblement à une distance par rapport au centre de rotation du genou. La partie postérieure du guide rigide inférieur maintenant cette première sangle est positionnée au niveau de la jambe selon un angle d'environ 65° par rapport à l'axe tibial, tandis qu'au niveau de la cuisse, la partie postérieure du guide rigide supérieur de la sangle est positionnée selon un angle d'environ 72° par rapport à l'axe fémoral.
- Une deuxième sangle est positionnée au niveau de la cuisse à environ 4 fois la distance de la première sangle par rapport au centre de rotation du genou et au niveau de la jambe, à environ 1,5 fois cette distance. La partie postérieure du guide rigide inférieur de cette deuxième sangle est positionnée, au niveau de la jambe, selon un angle d'environ 48° par rapport à l'axe tibial et, au niveau de la cuisse, la partie postérieure du guide rigide supérieur de la sangle est positionnée selon un angle d'environ 40° par rapport à l'axe fémoral.
- Une troisième sangle est positionnée au niveau de la cuisse et de la jambe à égales distances entre la deuxième sangle et une quatrième sangle. La partie postérieure du guide rigide inférieur de cette troisième sangle, est positionnée, au niveau de la jambe, selon un angle d'environ 39° par rapport à l'axe tibial, tandis qu'au niveau de la cuisse, la partie postérieure du guide rigide supérieur de la sangle est positionnée, selon un angle d'environ 40° par rapport à l'axe fémoral. La partie postérieure du guide rigide inférieur de la quatrième sangle est positionnée, au niveau de la jambe, selon un angle d'environ 35° par rapport à l'axe tibial, tandis qu'au niveau de la cuisse, la partie postérieure du guide rigide supérieur est positionnée selon un angle d'environ 40° par rapport à l'axe fémoral ;

Selon d'autres caractéristiques :
- au niveau de la jambe, les extrémités postérieures des guides rigides inférieurs des deuxième, troisième et quatrième sangles sont situées sur une droite faisant un angle d'environ 20° par rapport à l'axe tibial, tandis que l'extrémité postérieure du guide rigide inférieur de la première sangle est située sur une droite formant un angle d'environ 15° par rapport à l'axe tibial.
- au niveau de la cuisse, les extrémités postérieures des deuxième, troisième et quatrième sangles sont situées sur une droite faisant un angle d'environ 20° par rapport à l'axe fémoral, tandis que l'extrémité postérieure de la première sangle est située sur une droite formant un angle d'environ 15° par rapport à l'axe fémoral.

Avantageusement, dans l'orthèse selon l'invention, à partir du creux poplité, les brins supérieurs et inférieurs de la première sangle, entre la jambe et la cuisse, sont orientés angulairement selon un angle aigu dont le sommet est représenté par leur croisement à la face postérieure du genou, tandis que les brins des autres sangles sont orientés angulairement selon un angle obtus dont le sommet est représenté par leur croisement à la face postérieure du genou.

Avantageusement, dans l'orthèse selon l'invention, la première sangle, considérée du côté antérieur au niveau du tibia, présente une partie rigide profilée apte à passer au-dessus du tendon rotulien, en appui sur le tibia de part et d'autre de ce dernier

Selon des modes de réalisations différents, dans l'orthèse selon l'invention, la totalité ou une partie des sangles est élastique ou la totalité des sangles, est non élastique.

Avantageusement et selon une autre caractéristique, une partie seulement des sangles est élastique. Dans ce cas, à partir du creux poplité, la première sangle n'est pas élastique, les autres sangles présentant une élasticité avec un degré d'allongement progressif.

Sans pour cela sortir du cadre de l'invention, on n'exclut pas que la totalité des sangles soit élastique ou non élastique.

### BREVE DESCRIPTION DES FIGURES

L'invention est exposée ci-après plus en détail à l'aide des figures des dessins annexés dans lesquels :
- la figure 1 est une vue de profil et à caractère schématique montrant le principe de l'orthèse selon l'invention en position de flexion ;
- la figure 2 est une vue de face correspondant à la figure 1 ;
- la figure 3 est une vue arrière correspondant à la figure 1 ;
- la figure 4 est une vue semblable à la figure 1 en position d'extension ;
- la figure 5 est une vue à caractère schématique montrant notamment le positionnement des sangles au niveau de la jambe et de la cuisse ;
- la figure 6 est une vue partielle montrant un exemple d'accouplement et de fixation réglable des extrémités de l'une des sangles par rapport à un élément support que présente une partie de l'armature ;
- la figure 7 montre le positionnement angulaire des sangles par rapport à la jambe et à la cuisse ;
- la figure 8 montre le principe de croisement des angles au niveau du creux poplité en vue postérieure ;
- la figure 9 est une vue en coupe transversale considérée selon la ligne 9-9 de la figure 8 ;
- la figure 10 est, à une échelle plus importante, une vue en coupe partielle passant par la partie inférieure et antérieure de la première sangle ;
- la figure 11 est une vue partielle en perspective à caractère schématique au niveau de la cuisse d'un exemple indicatif nullement limitatif d'une structure d'orthèse assujettie aux sangles de contention selon les caractéristiques de l'invention.

### DESCRIPTION DETAILLEE D'UN MODE DE REALISATION PREFERE DE L'INVENTION

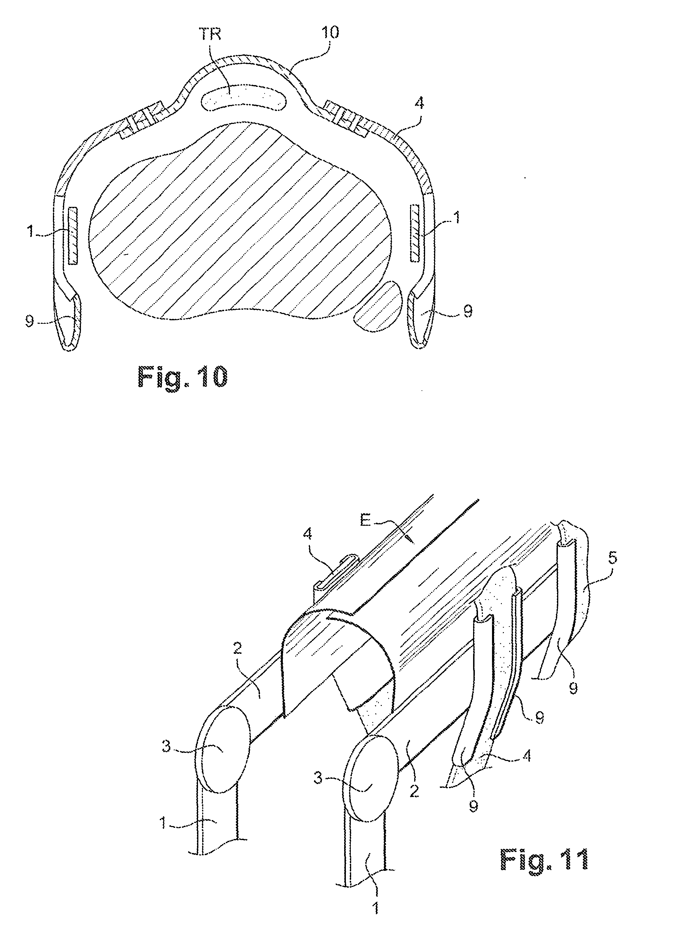

On a illustré, figure 11, un exemple indicatif nullement limitatif d'une armature pour orthèse du genou. Par exemple, cette armature comprend symétriquement deux bras (1) et (2) articulés en (3), au niveau sensiblement du centre de rotation du genou. En effet, de manière parfaitement connue, les deux bras (1) et (2) sont disposés de part et d'autre de la jambe et de la cuisse, en étant maintenus par tout moyen connu et approprié, telles que des enveloppes en tissu (E) présentant des moyens d'accouplement temporaire, du type ruban auto-agrippant par exemple.

Selon une caractéristique à la base de l'invention, cette armature, quelle que soit sa forme de réalisation, est assujettie à des moyens de contention sous forme de sangles (4), (5), (6) et (7) préalablement positionnées et orientées, comme il sera défini dans la suite de la description, pour entourer la jambe et la cuisse, en laissant libre la zone rotulienne, en étant croisées et superposées du côté postérieur à la surface cutané du creux poplité, au niveau du centre de rotation du genou avec, pour fonction, d'éviter à la partie antérieure et supérieure du tibia de glisser vers l'avant. Les sangles (4), (5), (6) et (7) sont maintenues dans des guides rigides (9) solidaires d'une partie de l'armature au niveau de la jambe et de la cuisse.

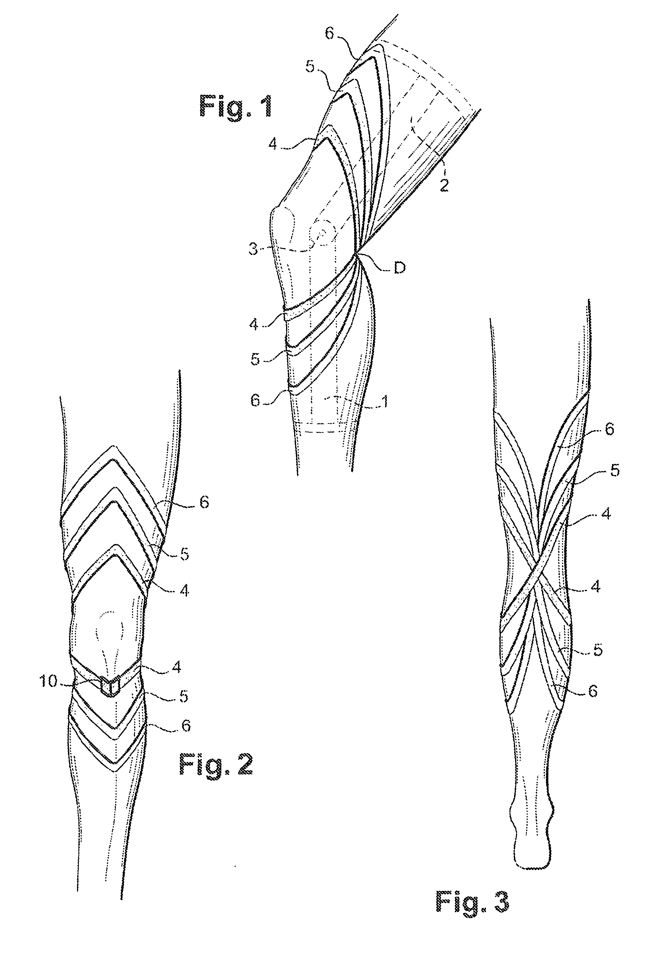

On renvoie notamment aux figures 1, 2, 3 et 4 qui montrent le positionnement de trois sangles (4), (5) et (6) en relation avec les bras (1) et (2) de l'armature.

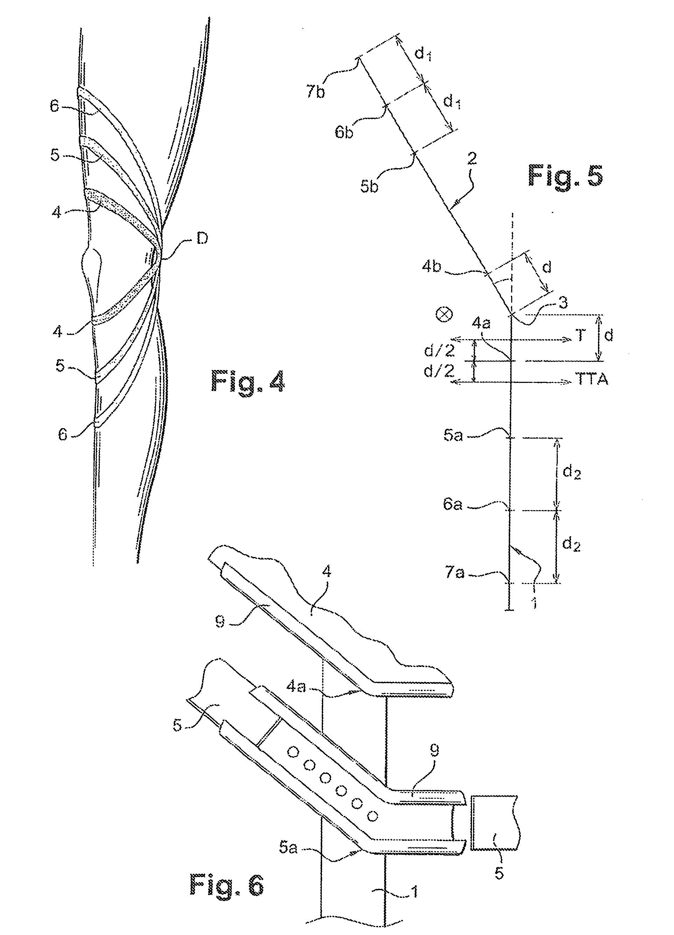

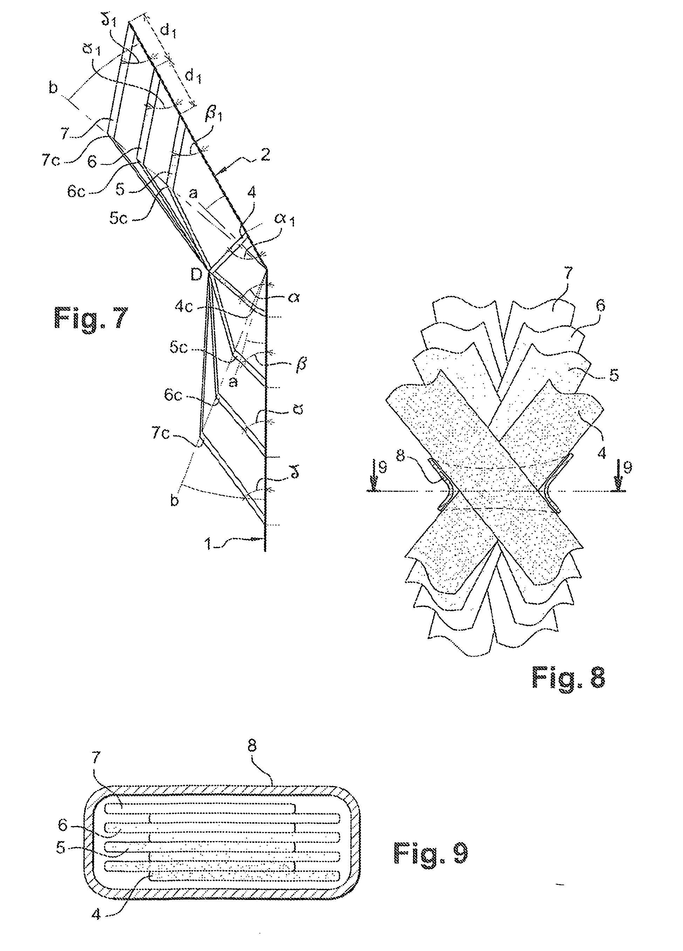

En considérant le problème posé à résoudre qui est de réduire le tiroir antérieur, autrement dit d'éviter à la partie antérieure et supérieure du tibia de glisser vers l'avant comme indiqué, le positionnement, l'orientation et le croisement des sangles, au niveau du creux poplité, sont avantageusement réalisés dans les conditions définies ci-après, en référence notamment aux vues schématiques figures 5 et 7. Sur ces figures, sont notamment montrés, très schématiquement, les bras (1), au niveau de la jambe, et les bras (2) au niveau de la cuisse et leur articulation en (3) correspondant sensiblement au centre de rotation du genou. Les guides rigides (9) pour le maintien et l'orientation angulaire des différentes sangles, ne sont pas représentés.

La première sangle (4) est positionnée en (4a) sur chaque bras (1) et, par rapport au centre de rotation (3), à égale distance (d/2) des plateaux tibiaux (T) et de la tubérosité tibiale antérieure (TTA) et en (4b) sur chaque bras (2), sensiblement à une distance (d) par rapport au centre de rotation (3). La partie postérieure des guides rigides de cette première sangle (4) est positionnée au niveau de chaque bras (1) selon un angle (α) d'environ 65° par rapport à l'axe tibial (bras (1),) tandis qu'au niveau de chaque bras (2), la partie postérieure du guide rigide est positionnée selon un angle (α1) d'environ 72° par rapport à l'axe fémoral (bras (2)) (figure 7).

La deuxième sangle (5) est positionnée en (5b) sur chaque bras (2) à environ 4 fois la distance (d) et positionnée en (5a) sur chaque bras (1) à environ 1,5 fois la distance (d). La partie postérieure du guide rigide de cette deuxième sangle (5) est positionnée au niveau de chaque bras (1), c'est-à-dire au niveau de la jambe, selon un angle (β) d'environ 48° par rapport à l'axe tibial et, au niveau de chaque bras (2) c'est-à-dire au niveau de la cuisse, selon un angle (β1) d'environ 40° par rapport à l'axe fémoral.

Dans l'exemple illustré aux figures 5 et 7, une troisième sangle (6) est positionnée en (6b) au niveau des bras (2), c'est-à-dire au niveau de la cuisse et en (6a) au niveau des bras (1), c'est-à-dire au niveau de la jambe, à égale distance ((d1) au niveau de la cuisse et (d2) au niveau de la jambe) entre les points d'attache (5b) et (5a), et ceux d'une quatrième sangle (7) fixée en (7b) au niveau de chaque bras (2) et en (7a) au niveau de chaque bras (1).

Les parties postérieures des guides rigides des troisième et quatrième sangles (6) et (7), sont respectivement positionnées au niveau de chaque bras (1) selon un angle (γ) et (δ) de 39° et 35° par rapport à l'axe tibial, et, au niveau de chaque bras (2), selon un angle (γ1) et (δ1) de 40° par rapport à l'axe fémoral.

Comme le montre la figure 7, l'extrémité postérieure (4c) du guide rigide de la première sangle (4), aussi bien au niveau de la jambe que de la cuisse, est située sur une droite (a) formant un angle d'environ 15° par rapport à l'axe tibial (bras (1)) ou par rapport à l'axe fémoral. Les extrémités postérieures (5c), (6c) et (7c) des guides rigides des autres sangles (5), (6) et (7) sont, quant à elles, situées au niveau de la jambe et de la cuisse, sur une droite (b) faisant un angle d'environ 20° par rapport aux axes tibial et fémoral.

A partir du creux de poplité (D), les brins de la première sangle (4), entre la jambe et la cuisse, sont orientés angulairement selon un angle aigu, tandis que les brins des autres sangles (5), (6) et (7) sont orientés angulairement selon un angle obtus. Autrement dit, les sangles (5), (6) et (7) ont un angle inversé par rapport à celui de la sangle (4), afin d'exercer une traction sur cette dernière, lors de l'extension du genou, évitant au plateau tibial de partir en avant, en cas de rupture du ligament croisé antérieur.

Au niveau de leur croisement postérieur au creux poplité (D), les sangles (4), (5), (6) et (7) sont maintenues ensemble par un manchon (8) non extensible. La première sangle (4) est disposée à l'extérieur, c'est-à-dire le plus en arrière possible (figures 8 et 9). La dernière sangle (7) (la plus longue) est disposée à l'intérieur, le plus en avant possible, en regard de la surface cutanée du creux poplité.

Selon une autre caractéristique et d'une manière avantageuse, la première sangle (4) est non élastique en ce sens qu'elle présente un allongement nul ou quasiment nul, tandis que les autres sangles (5), (6) et (7) présente une élasticité avec un degré d'allongement progressif.

Bien évidemment, les différentes zones d'accouplement et de positionnement (4a) - (4b), (5a) - (5b), (6a) - (6b) et (7a) - (7b), sur chacun des bras (1) et (2) de l'armature, dans les conditions indiquées précédemment, s'effectuent par tout moyen connu. Par exemple, les sangles (4), (5), (6) et (7) sont emmanchées séparément, avec capacité de réglage par coulissement et de blocage en position, dans les guides rigides (9) disposés aux différentes zones précitées des bras (1) et (2).

A noter également, comme le montre la figure 10, qu'une partie de la sangle (4), considérée du côté antérieur au niveau du tibia, est assujettie à une partie rigide (10) profilée sensiblement en forme d'arceau ou oméga pour passer au-dessus du tendon rotulien (TR), en appui de part et d'autre de ce dernier.

Les avantages ressortent bien de la description, en particulier on souligne et on rappelle que le système de contention par sangles en considérant leur positionnement et leur croisement, empêche la partie antérieure et supérieure du tibia de glisser vers l'avant (tiroir antérieur), en cas de rupture complète du ligament croisé antérieur.

Enfin, on rappelle, comme déjà indiqué, que le nombre de sangles peut être variable en fonction de la taille et de la musculature du patient, et de l'effet recherché. Il en est de même en ce qui concerne les différents degrés d'allongement des sangles, lesquelles peuvent être non élastiques.

Enfin, le système de sangles peut être assujetti à tout type d'armature parfaitement connue présentant une partie antérieure rigide.

## Revendications

1. Orthèse du genou pour rupture du ligament croisé antérieur comprenant une armature articulée (1) - (2) agencée pour être fixée au niveau de la cuisse et de la jambe, de part et d'autre du genou, ladite orthèse comprenant des moyens de contention sous forme d'une pluralité de sangles (4, 5, 6, 7) accouplées à ladite armature et aptes à entourer la cuisse et la jambe en laissant libre la zone rotulienne et à être croisées et superposées du côté postérieur en regard de la surface cutanée du creux poplité (D) au même niveau que le centre de rotation du genou afin d'éviter à la partie antérieure du tibia de glisser vers l'avant, **caractérisée en ce que** chaque sangle (4, 5, 6, 7) est maintenue dans un guide rigide (9) solidaire d'une partie de l'armature au niveau de la jambe et de la cuisse et apte à assurer le maintien et l'orientation angulaire desdites sangles, tandis qu'au niveau de leur croisement au creux poplité (D), les sangles (4, 5, 6, 7) sont maintenues ensembles par un manchon (8) inextensible, la première sangle 4, la plus courte, étant disposée à l'extérieur, le plus en arrière possible et la dernière sangle (7), la plus longue, étant disposée à l'intérieur, le plus en avant possible en regard de la surface cutanée du creux poplité.

2. Orthèse selon la revendication 1, **caractérisée en ce qu'**une première sangle (4) est positionnée au niveau de la jambe et par rapport au centre de rotation du genou, à égale distance (d/2) du plateau tibial (T) et de la tubérosité tibiale antérieure (TTA) et, au niveau de la cuisse, sensiblement à une distance (d) par rapport au centre de rotation du genou, la partie postérieure du guide rigide (9) maintenant la première sangle (4), étant positionnée au niveau de la jambe selon un angle d'environ 65° par rapport à l'axe tibial et, au niveau de la cuisse, selon un angle d'environ 72° par rapport à l'axe fémoral.

3. Orthèse selon la revendication 2, **caractérisée en ce qu'**une deuxième sangle (5) est positionnée au niveau de la cuisse à environ 4 fois la distance (d) de la première sangle (4) par rapport au centre de rotation du genou et, au niveau de la jambe, à environ 1,5 fois cette distance (d), la partie postérieure du guide rigide (9) de la deuxième sangle (5) étant positionnée, au niveau de la jambe, selon un angle d'environ 48° par rapport à l'axe tibial et, au niveau de la cuisse, selon un angle d'environ 40° par rapport à l'axe fémoral.

4. Orthèse selon la revendication 3, **caractérisée en ce qu'**une troisième sangle (6) est positionnée, au niveau de la cuisse et de la jambe, à égales distances (d1) et (d2) entre la deuxième sangle (5) et une quatrième sangle (7), la partie postérieure du guide rigide (9) de la troisième sangle (6) étant positionnée, au niveau de la jambe, selon un angle d'environ 39° par rapport à l'axe tibial et, au niveau de la cuisse, selon un angle d'environ 40° par rapport à l'axe fémoral.

5. Orthèse selon la revendication 4, **caractérisée en ce que** la partie postérieure du guide rigide de la quatrième sangle (7) est positionnée, au niveau de la jambe, selon un angle d'environ 35° par rapport à l'axe tibial et, au niveau de la cuisse, selon un angle d'environ 40° par rapport à l'axe fémoral.

6. Orthèse selon l'une des revendications 4 à 5, **caractérisée en ce qu'**au niveau de la jambe et de la cuisse, les extrémités postérieures (5c), (6c) et (7c) des guides rigides des deuxième, troisième et quatrième sangles (5, 6, 7) sont situées sur une droite faisant un angle d'environ 20° par rapport à l'axe fémoral ou à l'axe tibial, tandis que l'extrémité postérieure (4c) de la première sangle (4) est située sur une droite formant un angle d'environ 15° par rapport à l'axe fémoral ou à l'axe tibial.

7. Orthèse selon l'une des revendications 1 - 6, **caractérisée en ce qu'**à partir du creux poplité (D), les brins supérieurs et inférieurs de la première sangle (4), entre la jambe et la cuisse, sont orientés angulairement selon un angle aigu dont le sommet est représenté par leur croisement à la face postérieure du genou, tandis que les brins des autres sangles (5, 6, 7) sont orientés angulairement selon un angle obtus dont le sommet est représenté par leur croisement à la face postérieure du genou.

8. Orthèse selon l'une des revendications 1 - 7, **caractérisée en ce que** la première sangle (4), considérée du côté antérieur au niveau du tibia, présente une partie rigide profilée (10) apte à passer au-dessus du tendon rotulien, en appui sur le tibia de part et d'autre de ce dernier.

9. Orthèse selon l'une des revendications 1 - 8, **caractérisée en ce que** la totalité ou une partie des sangles (4, 5, 6, 7) est élastique.

10. Orthèse selon l'une des revendications 1 - 8, **caractérisée en ce que** la totalité des sangles (4, 5, 6, 7) est non élastique.

11. Orthèse selon la revendication 9, **caractérisée en ce que** la première sangle (4) n'est pas élastique, les autres sangles (5, 6, 7) présentant une élasticité avec un degré d'allongement progressif.

12. Orthèse selon l'une des revendications 1 - 1 1, **caractérisée en ce que** les extrémités des sangles (4, 5, 6, 7) sont engagées séparément avec capacité de réglage par coulissement et de blocage en position, dans les guides rigides (9) rendus solidaires d'une partie de l'armature, au niveau de la cuisse et de la jambe.

## Patentansprüche

1. Orthese des Knies wegen eines Risses des vorderen Kreuzbandes, umfassend eine artikulierte Armatur (1) - (2), die angeordnet ist, um am Oberschenkel und am Unterschenkel auf jeder Seite des Knies befestigt zu werden, wobei die genannte Orthese Kompressionsmittel in Form einer Vielzahl von Gurten (4, 5, 6, 7) umfasst, die an die genannte Armatur gekoppelt und geeignet sind, den Oberschenkel und den Unterschenkel zu umschließen und den Kniescheibenbereich frei zu lassen und auf der hinteren Seite gegenüber der Hautfläche der Kniekehle (D) auf derselben Höhe wie des Rotationszentrums des Knies gekreuzt und überlagert zu werden, um den vorderen Teil des Schienbeins daran zu hindern, nach vorne zu rutschen, **dadurch gekennzeichnet, dass** jeder Gurt (4, 5, 6, 7) in einer steifen Führung (9) festgehalten ist, die mit einem Teil der Armatur am Unterschenkel und dem Oberschenkel fest verbunden ist und geeignet ist, den Halt und die winkelförmige Ausrichtung der genannten Gurte zu gewährleisten, während die Gurte (4, 5, 6, 7) an ihrer Kreuzung in der Kniekehle (D) zusammen durch eine nicht dehnbare Halterung gehalten werden, wobei der erste und kürzere Gurt (4) an der Außenseite so weit hinten wie möglich angeordnet ist und der letzte und längere Gurt (7) an der Innenseite so weit vorne wie möglich gegenüber der Hautfläche der Kniekehle angeordnet ist.

2. Orthese gemäß Anspruch 1, **dadurch gekennzeichnet, dass** ein erster Gurt (4) am Unterschenkel und im Verhältnis zum Rotatioriszentrum des Knies in der gleichen Entfernung (d/2) von der Schienbeinplatte (T) und des vorderen Schienbeinhöckers (TTA) und am Oberschenkel deutlich in einer Entfernung (d) im Verhältnis zum Rotationszentrum des Knies positioniert ist, wobei der hintere Teil der steifen Führung (9), der den ersten Gurt (4) hält, am Unterschenkel gemäß einem Winkel von rund 65° im Verhältnis zur Schienbeinachse und am Oberschenkel gemäß einem Winkel von rund 72° im Verhältnis zur Oberschenkelachse positioniert ist.

3. Orthese gemäß Anspruch 2, **dadurch gekennzeichnet, dass** ein zweiter Gurt (5) am Oberschenkel ungefähr in der 4-fachen Entfernung (d) vom ersten Gurt (4) im Verhältnis zum Rotationszentrum des Knies und am Unterschenkel ungefähr in der 1,5-fachen dieser Entfernung (d) positioniert ist, wobei der hintere Teil der steifen Führung (9) des zweiten Gurts (5) am Unterschenkel gemäß einem Winkel von ungefähr 48° im Verhältnis zur Schienbeinachse und am Oberschenkel gemäß einem Winkel von rund 40° im Verhältnis zur Oberschenkelachse positioniert ist.

4. Orthese gemäß Anspruch 3, **dadurch gekennzeichnet, dass** ein dritter Gurt (6) am Oberschenkel und am Unterschenkel in gleichen Entfernungen (d1) und (d2) zwischen dem zweiten Gurt (5) und einem vierten Gurt (7) positioniert sind, wobei der hintere Teil der steifen Führung (9) des dritten Gurts (6) am Unterschenkel gemäß einem Winkel von rund 39° im Verhältnis zur Schienbeinachse und am Oberschenkel gemäß einem Winkel von rund 40° im Verhältnis zur Oberschenkelachse positioniert ist.

5. Orthese gemäß Anspruch 4, **dadurch gekennzeichnet, dass** der hintere Teil der steifen Führung des vierten Gurts (7) am Oberschenkel gemäß einem Winkel von rund 35° im Verhältnis zur Schienbeinachse und am Oberschenkel gemäß einem Winkel von rund 40° im Verhältnis zur Oberschenkelachse positioniert ist.

6. Orthese gemäß Anspruch 4 bis 5, **dadurch gekennzeichnet, dass** sich die hinteren Enden (5c), (6c) und (7c) der steifen Führungen des zweiten, dritten und vierten Gurtes (5, 6, 7) am Unterschenkel und am Oberschenkel auf einer Gerade befinden, die einen Winkel von rund 20° im Verhältnis zur Oberschenkelachse oder zur Schienbeinachse bilden, während sich das hintere Ende (4c) des ersten Gurtes (4) auf einer Geraden befindet, die einen Winkel von rund 15° im Verhältnis zur Oberschenkelachse oder zur Schienbeinachse bildet.

7. Orthese gemäß Anspruch 1 bis 6, **dadurch gekennzeichnet, dass** die oberen und unteren Faser des ersten Gurtes (4) zwischen dem Unterschenkel und dem Oberschenkel ab der Kniekehle (D) winkelförmig gemäß einem spitzen Winkel ausgerichtet sind, dessen Spitze durch ihre Kreuzung an der hinteren Seite des Knies dargestellt ist, während die Faser der anderen Gurte (5, 6, 7) winkelförmig gemäß einem stumpfen Winkel ausgerichtet sind, dessen Spitze durch ihre Kreuzung an der hinteren Seiten des Knies dargestellt ist.

8. Orthese gemäß Anspruch 1 - 7, **dadurch gekennzeichnet, dass** der erste Gurt (4) von der vorderen Seite auf Höhe des Schienbeins betrachtet einen profilierten steifen Teil (10) aufweist, der geeignet ist, über der Kniesehne aufstützend auf dem Schienbein auf jeder Seite desselben vorbeizulaufen.

9. Orthese gemäß Anspruch 1 - 8, **dadurch gekennzeichnet, dass** alle oder ein Teil der Gurte (4, 5, 6, 7) elastisch sind / ist.

10. Orthese gemäß Anspruch 1 - 8, **dadurch gekennzeichnet, dass** keiner der Gurte (4, 5, 6, 7) elastisch ist.

11. Orthese gemäß Anspruch 9, dadurch gekenntzeichnet, dass der erste Gurt (4) nicht elastisch ist, wobei die anderen Gurte (5, 6, 7) eine Elastizität mit einem progressiven Verlängerungsgrad aufweisen.

12. Orthese gemäß Anspruch 1-11, **dadurch gekennzeichnet, dass** die Enden der Gurte (4, 5, 6, 7) getrennt mit einer Einstellkapazität per Gleiten und Verrasten in der Position in den fest befestigten steifen Führungen (9) eines Teils der Armatur am Oberschenkel und am Unterschenkel eingegriffen sind.

## Claims

1. Knee orthosis for torm anterior cruciate ligament tears comprising articulated annature (1) - (2) arranged to be attached to the thigh and leg, on either side of the knee, said orthosis comprising means of restraint in the form of a plurality of straps (4, 5, 6, 7) coupled to the said armature and adapted to surround the thigh and leg leaving free the patellar area and to be crossed and superimposied on the posterior side facing the skin of the popliteal fossa (D) at the same level of the center of rotation of the knee in order to avoid the anterior part of the tibia sliding forward, **characterised in that** each strap (4, 5, 6, 7) is held in a rigid guide (9) integral with a part of the armature, at the leg and thigh, and adapted to maintain the angular orientation of said straps, while where they cross in the popliteal fossa (D), the straps (4, 5, 6, 7) are held together by a non-expandable sleeve (8), the first strap (4), the shortest, being disposed on the outside, as far backward as possible and the last strap (7), the longest, being disposed inside, as far forward as possible facing the skin of the popliteal fossa.

2. Orthosis according to claim 1, **characterised in that** a first strap (4) is positioned on the lower leg and relative to the center of rotation of the knee, at an equal distance (d/2) from the tibial plate (T) and the anterior tibial tuberosity (ATT) and, on the thigh, approximately at a distance (d) relative to the center of rotation of the knee, the posterior part of the rigid guide (9) holding the first strap (4) being positioned on the leg at an angle of approximately 65° to the tibial axis and, on the thigh, at an angle of approximately 72° to the femoral axis.

3. Orthosis according to claim 2, **characterised in that** a second strap (5) is positioned on the thigh at approximately 4 times the distance (d) of the first strap (4) from the center of rotation of the knee and, on the leg, at approximately 1.5 times this distance (d), the posterior part of the rigid guide (9) of the second strap (5) being positioned on the leg at an angle of approximately 48° to the tibial axis and on the thigh at an angle of approximately 40° to the femoral axis.

4. Orthosis according to claim 3, **characterised in that** a third strap (6) is positioned on the thigh and leg at equal distances (d1) and (d2) between the second strap (5) and a fourth strap (7), the posterior part of the rigid guide (9) of the third strap (6) being positioned on the lower leg at an angle of approximately 39° to the tibial axis and on the thigh at an angle of approximately 40° to the femoral axis.

5. Orthosis according to claim 4, **characterised in that** the posterior part of the rigid guide of the fourth strap (7) is positioned on the lower leg at an angle of approximately 35° to the tibial axis and on the thigh at an angle of approximately 40° to the femoral axis.

6. Orthosis according to one of claims 4 to 5, **characterised in that**, on the thigh and lower leg, the posterior ends (5c), (6c) and (7c) of the rigid guides of the second, third and fourth straps (5, 6, 7) are positioned on a straight line forming an angle of approximately 20° relative to the femoral or tibial axis, while the posterior end (4c) of the first strap (4) is positioned on a straight line forming an angle of approximately 15° relative the femoral or tibial axis.

7. Orthosis according to one of claims 1 - 6, **characterised in that** from the popliteal fossa (D,) the upper and lower strands of the first strap (4), between the leg and thigh, are regularly oriented at an acute angle, the summit of which is where they cross at the back of the knee, while the strands of the other straps (5, 6, 7) are regularly oriented at an obtuse angle, the summit of which being where they cross at the back of the knee.

8. Orthosis according to one of claims 1 - 7, **characterised in that** the first strap (4), on the anterior side on the tibia, has a shaped rigid part (10) adapted for passing over the patellar tendon, bearing on the tibia on either side of the latter.

9. Orthosis according to one of claims 1 - 8, **characterised in that** all or part of the straps (4, 5, 6, 7) are elastic.

10. Orthosis according to one of claims 1 - 8, **characterised in that** all the straps (4, 5, 6, 7) are non-elastic.

11. Orthosis according to claim 9, **characterised in that** the first strap (4) is not elastic, the other straps (5, 6, 7) being elastic with a progressive degree of lengthening.

12. Orthosis according to one of claims 1 - 11, **characterised in that** the ends the straps (4, 5, 6, 7) are engaged separately with adjustability by sliding and with locking ability in position, in the rigid guides (9) which are firmly attached to a part of the armature, on the thigh and leg.
